# EUROPEAN PATENT APPLICATION

(11) **EP 3 875 089 A1**
(43) Date of publication of application: **08.09.2021**
(21) Application number: 19880458.5
(22) Date of filing: 31.10.2019
(51) Int. Cl.: A61K 31/505, A61K 9/20, A61K 47/12, A61K 47/26, A61K 47/32, A61P 35/00, A61P 35/02

(54) **TABLET CONTAINING ANTITUMOR AGENT**

(30) Priority: 31.10.2018 JP 2018204852
(71) Applicant: FUJIFILM Corporation, Tokyo 106-8620 (JP)
(72) Inventor: OURA Tai, Toyama-shi, Toyama 930-8508 (JP)
(74) Representative: Hoffmann Eitle
(86) International application number: PCT/JP2019/042751
(87) International publication number: WO 2020/090971

(57) **Abstract**

An object of the present invention is to provide a tablet containing a succinate salt of (S,E)-N-(1-((5-(2-((4-cyanophenyl)amino)-4-(propylamino)pyrimidin-5-yl)-pent-4-yn-1-yl)am ino)-1-oxopropan-2-yl)-4-(dimethylamino)-N-methylbut-2-enamide (Compound A) which is rapidly dissolved in a weakly acidic solution in order to prevent a decrease in bioavailability. According to the present invention, there is provided a tablet containing a succinate salt of (S,E)-N-(1-((5-(2-((4-cyanophenyl)amino)-4-(propylamino)pyrimidin-5-yl)-pent-4-yn-1-yl)am ino)-1-oxopropan-2-yl)-4-(dimethylamino)-N-methylbut-2-enamide (Compound A), at least one or more kinds selected from the group consisting of sugars and sugar alcohol, and crospovidone.

## Description

### Field of the Invention

The present invention relates to a tablet exhibiting an excellent dissolution property, which contains a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide (hereinafter, referred to as Compound A).

### Description of the Related Art

Compound A is a medically useful compound that has an FLT3 inhibitory action and has a strong effect on hematological cancer such as acute myeloid leukemia (Patent Document 1). In the medical field, there is a demand on a highly effective and stable pharmaceutical drug, which is expected to be applied clinically in a wide range of administration formulation forms such as an oral agent, an injection agent, an ointment, and a suppository.

On the other hand, with the advancement of in-home medical care in recent years, the promotion of self-medication has been advocated, the maintenance of medication adherence is an important issue in drug therapy. The most commonly used formulation form in-home medical care is an oral agent (Non-Patent Document 1), and the most preferred formulation form for patients is generally a tablet (Non-Patent Document 2).

In a case where a formulation is orally administered, the formulation first enters the stomach and then in the process of migrating from the stomach to the small intestine, disintegrates and disperses, and the drug contained in the formulation becomes dissolved. The average pH in the stomach temporarily rises to around pH 4 at the time of having a meal during the day; however, the pH drops after about 30 minutes from the time having a meal and a low pH of around pH 2 is exhibited between meals (Non-Patent Document 3). On the other hand, it has been considered that in general, the acidity of gastric juice in healthy adults is high and is about pH 1; however, it has been clear that the proportion of adults whose gastric acid secretion is reduced increases as the age increases (Non-Patent Document 4). That is, the dissolution property not only in an acidic test solution but also in a weakly acidic test solution is one of the important indicators of a formulation. Generally, a formulation in which 85% or more of a drug is dissolved within 30 minutes is considered to have a rapid dissolution property.

### Prior Art Documents

### Patent Documents

Patent Document 1: WO2015/056683A

### Non-Patent Documents

Non-Patent Document 1: Japanese Journal of Pharmaceutical Health Care and Sciences, Vol. 34, No. 3, pp. 289 to 296, 2008
Non-Patent Document 2: CLINICIAN, Vol. 38, No. 10, pp. 1019 to 1022, 1991
Non-Patent Document 3: Journal of Japanese Society of Gastroenterology, Vol. 85, No. 7, pp. 1353 to 1359, 1988
Non-Patent Document 4: Bioequivalence test of pharmaceutical products - Guideline compliance -, edited by Hiroyasu Ogata, p. 35, 2013

### SUMMARY OF THE INVENTION

The tablet produced by a conventional method containing the succinate salt of Compound A showed a low dissolution property in a dissolution test with a test solution having a pH of 4.5, where the test solution was a weakly acidic test solution.

In order to prevent a decrease in bioavailability, a tablet containing a succinate salt of Compound A that is rapidly dissolved in a weakly acidic solution is strongly desired.

Under such circumstances, the inventors of the present invention carried out extensive studies and have found that a tablet containing specific sugars or specific sugar alcohol, and crospovidone has an effect of improving dissolution in a weakly acidic solution. Furthermore, the inventors of the present invention have found that the tablet has a strength with which cracking and chipping do not occur during the manufacturing process or transportation.

The present invention provides a tablet containing a succinate salt of Compound A, having a rapid dissolution property, and having a sufficient strength.

The present invention provides the followings.
<1> A tablet comprising a succinate salt of (S,E)-N-(1-((5-(2-((4-cyanophenyl)amino)-4-(propylamino)pyrimidin-5-yl)-pent-4-yn-1-yl)am ino)-1-oxopropan-2-yl)-4-(dimethylamino)-N-methylbut-2-enamide, at least one or more kinds selected from the group consisting of sugars and sugar alcohol, and crospovidone.
<2> The tablet according to <1>, in which the sugars are lactose and the sugar alcohol is erythritol.
<3> The tablet according to <1> or <2>, further comprising a lubricant.
<4> The tablet according to <3>, in which the lubricant is magnesium stearate or sodium stearyl fumarate.

The tablet containing a succinate salt of Compound A according to an aspect of the present invention is a tablet having an excellent dissolution property and having a strength required in the manufacturing process and the transportation process.

### DESCRIPTION OF THE PREFERRED EMBODIMENTS

The present invention will be described in detail below.

As used in the present specification, % means the mass percentage unless otherwise particularly specified. In the present invention, the numerical range indicated by using "to" indicates a range including numerical values described before and after "to" as a minimum value and a maximum value, respectively. In addition, in the present invention, in a case where there are a plurality of substances corresponding to each of components in a tablet, the amount of each of components in the tablet means the total amount of the plurality of substances present in the tablet unless otherwise particularly specified.

A succinate salt of Compound A used in the present invention can be produced, for example, by the method disclosed in WO2015/056683A.

The tablet according to the embodiment of the present invention is a tablet containing a succinate salt of Compound A, at least one of the group consisting of sugars and sugar alcohol, and crospovidone.

The tablet according to the embodiment of the present invention has an excellent dissolution property; for example, in the dissolution test of the 17th revised Japanese Pharmacopoeia dissolution test method (the paddle method), the dissolution rate after 30 minutes is 85% by mass or more, where the dissolution test is carried out using an acetate buffer solution having a pH of 4.5 as the test solution under the condition of the rotation speed of 50 rotations/minute.

The tablet according to the embodiment of the present invention has a high hardness, for example, a hardness of 50 N or more.

Examples of the sugars which are used in the present invention include glucose, sucrose, lactose, maltose, and trehalose hydrate, preferred examples thereof include lactose, maltose, and trehalose hydrate, and more preferred examples thereof include lactose.

Examples of the lactose include hydrated lactose, unhydrated lactose, a spray-dried hydrated lactose, and a granulated hydrated lactose, and preferred examples thereof include a spray-dried hydrated lactose.

Examples of the sugar alcohol which is used in the present invention include erythritol, sorbitol, lactitol, isomalt, and xylitol, preferred examples thereof include erythritol and sorbitol, and more preferred examples thereof include erythritol.

The sugars and the sugar alcohol may be used alone or in a combination of two or more thereof.

The total content of sugars and sugar alcohol may be 5% to 95%, preferably 10% to 80%, and more preferably 15% to 75%, with respect to the tablet.

Examples of the crospovidone which is used in the present invention include an A type and a B type, which are classified according to the particle size.

The content of crospovidone may be 0.1% to 30%, preferably 0.5% to 20%, and more preferably 1% to 15%, with respect to the tablet.

In the tablet according to the embodiment of the present invention, a pharmaceutically acceptable pharmaceutical aid can be used within the range in which the effects of the present invention are not impaired.

Examples of the pharmaceutical aid include a binder, a lubricant, a sweetening agent, a coloring agent, a flavoring agent, a surfactant, a coating agent, and a plasticizer.

Examples of the binder include hydroxypropyl cellulose, carmellose sodium, and methyl cellulose, hypromellose, and polyvinyl alcohol.

Examples of the lubricant include stearic acid, magnesium stearate, calcium stearate, sodium stearyl fumarate, talc, hydrated silicon dioxide, light anhydrated silicic acid, and sucrose esters of fatty acids. Preferred examples thereof are magnesium stearate and sodium stearyl fumarate.

Examples of the sweetening agent include aspartame, saccharin, stevia, thaumatin, and acesulfame potassium.

Examples of the colorant include titanium dioxide, red ferric oxide, yellow ferric oxide, black iron oxide, Food Red No. 102, Food Yellow No. 4, and Food Yellow No. 5.

Examples of the flavoring agent include essential oils such as orange oil, lemon oil, peppermint oil, and pine oil; extracts such as orange extract and peppermint extract; flavors such as cherry flavor, vanilla flavor, and fruit flavor; powdered fragrances such as apple micron, banana micron, peach micron, strawberry micron, and orange micron; vanillin; and ethyl vanillin.

Examples of the surfactant include sodium lauryl sulfate, sodium dioctyl sulfosuccinate, polysorbate, and polyoxyethylene-hardened castor oil.

Examples of the coating agent include hypromellose, an aminoalkyl methacrylate copolymer E, an aminoalkyl methacrylate copolymer RS, ethyl cellulose, cellulose acetate phthalate, hydroxypropyl methyl cellulose phthalate, a methacrylic acid copolymer L, a methacrylic acid copolymer LD, and a methacrylic acid copolymer S.

Examples of the plasticizer include triethyl citrate, macrogol, triacetin, and propylene glycol.

These additives may be used alone or in a combination of two or more thereof.

The blending amounts of these additives are not particularly limited, and blending may be carried out appropriately so that the effect thereof is sufficiently exhibited according to each purpose.

The method for producing the tablet according to the embodiment of the present invention is not particularly limited and may be carried out by a conventional method.

Examples of the method for producing the tablet according to the embodiment of the present invention include a wet-type granule compression method, a dry-type granule compression method, and a direct powder compression method, and a method in which these are combined, preferred examples thereof include a wet-type granule compression method and a dry-type granule compression method, and more preferred examples thereof include a dry-type granule compression method.

Examples of the wet-type granule compression method include a method in which a granulated product is produced by the wet-type granulation, one or more additives among an excipient, a disintegrant, and a lubricant are added to the obtained granulated product, the resultant mixture is mixed to produce a mixed powder, and the obtained mixed powder is tableted to obtain an uncoated tablet.

Examples of the wet-type granulation method include a fluidized bed type granulation method, a wet-type crushing granulation method, a rotary granulation method, a spray-drying type granulation method, and an extrusion granulation method, and preferred examples thereof include a fluidized bed type granulation method and the wet-type crushing granulation method.

Examples of the dry-type granule compression method include a method in which compression is carried out by the dry-type granulation method to produce a compression-molded product, the obtained compression-molded product is crushed and sized to produce a granulated product, one or more additives among an excipient, a disintegrant, and a lubricant are added to the obtained granulated product, the resultant mixture is mixed to produce a mixed powder, and the obtained mixed powder is tableted to obtain an uncoated tablet.

The uncoated tablet obtained by these production methods can also be film-coated.

Examples of the dry-type granulation method include a compacting method, a slugging method, and a briquetting method, and preferred examples thereof include a compacting method. Examples of the compacting method include a method of using a roller compactor.

Examples of the compression method (the tableting method) include a rotary tableting method, a single-punching tableting method, and an intermittent tableting method, and preferred examples thereof include a rotary tableting method.

### Examples

The usefulness of the tablet according to the embodiment of the present invention will be described in Examples and Comparative Examples; however, the present invention is not limited thereto.

In Examples and Comparative Examples, as a desktop V-type mixer, VM-2 manufactured by TSUTSUI Scientific Instruments Co., Ltd. was used;
as a dry-type granulator, TF-LABO manufactured by Freund Corporation was used;
as a rotary tableting machine, PICCOLA, B-10 manufactured by RIVA S.A. was used;
as sodium stearyl fumarate, PRUV manufactured by JRS Pharma was used;
as magnesium stearate, Parteck™ LUB MST manufactured by Merck KGaA was used; and
as lactose, SuperTab 11SD manufactured by DFE Pharma was used;
as erythritol, Erythritol T fine powder manufactured by Mitsubishi-Chemical Foods Corporation was used;
as mannitol, Parteck M100 manufactured by Merck KGaA was used; and
as crospovidone, Kollidon CL-SF manufactured by BASF SE was used.

### Example 1

10 g of a succinate salt of Compound A, 16.1 g of lactose, 0.8 g of crospovidone, and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Example 2

10 g of a succinate salt of Compound A, 15.5 g of lactose, 1.4 g of crospovidone, and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Example 3

10 g of a succinate salt of Compound A, 14.1 g of lactose, 2.7 g of crospovidone, and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Example 4

10 g of a succinate salt of Compound A, 15.5 g of lactose, 1.4 g of crospovidone, and 0.3 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Sodium stearyl fumarate was added to the obtained granulated product so that the content thereof was 0.5% of the total amount, and the resultant mixture was mixed with the desktop V-type mixer for 30 minutes. This mixed powder was tableted with a rotary tableting machine using a punch having a diameter of 8.5 mm at a tableting pressure of 10 kN so that the weight per tablet was 250 mg, whereby an uncoated tablet was obtained.

### Example 5

10 g of a succinate salt of Compound A, 15.5 g of erythritol, 1.4 g of crospovidone, and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 1

10 g of a succinate salt of Compound A, 15.5 g of lactose, 1.4 g of carmellose calcium (ECG505, manufactured by Nichirin Chemical Industries, Ltd.), and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 2

10 g of a succinate salt of Compound A, 15.5 g of lactose, 1.4 g of croscarmellose sodium (Ac-Di-Sol, manufactured by FMC Corporation), and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 3

10 g of a succinate salt of Compound A, 15.5 g of lactose, 1.4 g of sodium starch glycolate (Primojel, manufactured by DFE Pharma), and 0.3 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 4

10 g of a succinate salt of Compound A, 15.5 g of lactose, 1.4 g of low-substituted hydroxypropylcellulose (L-HPC LH-21, manufactured by Shin-Etsu Chemical Co., Ltd.), and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 5

10 g of a succinate salt of Compound A, 15.5 g of mannitol, 1.4 g of crospovidone, and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 6

10 g of a succinate salt of Compound A, 15.5 g of mannitol, 1.4 g of carmellose calcium (ECG505, manufactured by Nichirin Chemical Industries, Ltd.), and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Comparative Example 7

10 g of a succinate salt of Compound A, 15.5 g of microcrystalline cellulose (CEOLUS PH101 manufactured by Asahi Kasei Corporation), 1.4 g of carmellose calcium (ECG505, manufactured by Nichirin Chemical Industries, Ltd.), and 0.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.5% of the total amount, and the resultant mixture was mixed with the desktop V-type mixer for 30 minutes. The obtained mixed powder was tableted with a rotary tableting machine using a punch having a diameter of 8.5 mm at a tableting pressure of 10 kN so that the weight per tablet was 250 mg, whereby an uncoated tablet was obtained.

### Test example 1

As samples, the tablets of Examples 1 to 5 and Comparative Examples 1 to 7 were used.

The dissolution test was carried out by the Japanese Pharmacopoeia dissolution test method (the paddle method). The rotation speed of the paddle was 50 rotations/minute. The sample contained in the sinker was put into 900 mL of an acetate buffer solution having a pH of 4.5, the test solution was collected 30 minutes later, and the dissolution rate (%) of Compound A was determined by the absorbance method.

The results are shown in Table 1 and Table 2.

**[Table 1]**

| Unit (mg) | Example 1 | Example 2 | Example 3 | Example 4 | Example 5 | Comparative Example 1 |
|---|---|---|---|---|---|---|
| Succinate of Compound A | 91.5 | 91.5 | 91.5 | 91.5 | 91.5 | 91.5 |
| Lactose | 146.9 | 141.9 | 129.4 | 141.9 | | 141.9 |
| Erythritol | | | | | 141.9 | |
| Mannitol | | | | | | |
| Microcrystalline cellulose | | | | | | |
| Crospovidone | 7.5 | 12.5 | 25.0 | 12.5 | 12.5 | |
| Carmellose calcium | | | | | | 12.5 |
| Carmellose sodium | | | | | | |
| Sodium starch glycolate | | | | | | |
| Low-substituted hydroxypropylcellulose | | | | | | |
| Magnesium stearate | 4.1 | 4.1 | 4.1 | | 4.1 | 4.1 |
| Sodium stearyl fumarate | | | | 4.1 | | |
| Total | 250.0 | 250.0 | 250.0 | 250.0 | 250.0 | 250.0 |
| Dissolution rate (%) | 95 | 98 | 90 | 103 | 95 | 66 |
| Hardness (N) | 91 | 81 | 54 | 84 | 56 | 87 |

**[Table 2]**

| Unit (mg) | Comparative Example 2 | Comparative Example 3 | Comparative Example 4 | Comparative Example 5 | Comparative Example 6 | Comparative Example 7 |
|---|---|---|---|---|---|---|
| Succinate of Compound A | 91.5 | 91.5 | 91.5 | 91.5 | 91.5 | 91.5 |
| Lactose | 141.9 | 141.9 | 141.9 | | | |
| Erythritol | | | | | | |
| Mannitol | | | | 141.9 | 141.9 | |
| Microcrystalline cellulose | | | | | | 141.9 |
| Crospovidone | | | | 12.5 | | |
| Carmellose calcium | | | | | 12.5 | 12.5 |
| Carmellose sodium | 12.5 | | | | | |
| Sodium starch glycolate | | 12.5 | | | | |
| Low-substituted hyroxypropylcellulose | | | 12.5 | | | |
| Magnesium stearate | 4.1 | 4.1 | 4.1 | 4.1 | 4.1 | 4.1 |
| Sodium stearyl fumarate | | | | | | |
| Total | 250.0 | 250.0 | 250.0 | 250.0 | 250.0 | 250.0 |
| Dissolution rate (%) | 33 | 61 | 61 | 56 | 41 | 57 |
| Hardness (N) | 84 | 78 | 87 | 83 | 162 | 126 |

The tablet containing a succinate salt of Compound A, at least one of the group consisting of sugars and sugar alcohol, and crospovidone exhibited an excellent dissolution property as compared with Comparative Examples.

In addition, the hardness of each of the tablets of Examples and Comparative Examples was sufficient.

The tablet according to the embodiment of the present invention is useful as a tablet having a rapid dissolution property, which contains a succinate salt of Compound A.

## Claims

1. A tablet comprising:
a succinate salt of (S,E)-N-(1-((5-(2-((4-cyanophenyl)amino)-4-(propylamino)pyrimidin-5-yl)-pent-4-yn-1-yl)am ino)-1-oxopropan-2-yl)-4-(dimethylamino)-N-methylbut-2-enamide;
at least one or more kinds selected from the group consisting of sugars and sugar alcohol; and
crospovidone.

2. The tablet according to claim 1, wherein the sugars are lactose, and the sugar alcohol is erythritol.

3. The tablet according to claim 1 or 2, further comprising a lubricant.

4. The tablet according to claim 3, wherein the lubricant is magnesium stearate or sodium stearyl fumarate.
